# EUROPEAN PATENT APPLICATION

(11) **EP 3 289 888 A1**
(43) Date of publication of application: **07.03.2018**
(21) Application number: 17382031.7
(22) Date of filing: 24.01.2017
(51) Int. Cl.: A23L 33/135, A61K 35/741, A61K 8/99

(54) **HEALTH PRODUCTS WITH INACTIVATED PROBIOTICS AND THEIR USES**

(30) Priority: 05.09.2016 ES 201631156
(71) Applicant: De La Fuente Blasco, Pedro Jose, 28022 Madrid (ES)
(72) Inventor: De La Fuente Blasco, Pedro Jose, 28022 Madrid (ES)
(74) Representative: González López-Menchero, Álvaro Luis

(57) **Abstract**

Health products do not meet the conditions in order for common probiotics to be preserved since they have a degree of moisture greater than 5% and/or a temperature greater than 5°C in any of the processes of production, storage and preservation and into which the inactivated probiotics have been incorporated. The inactivation of the probiotics is achieved by means of subjecting the probiotic strains to high temperatures (range 50°C to 140°C) for a time period of between 30 seconds and 90 minutes depending on the time of exposure of the bacteria to temperatures, in various consecutive sessions and with subsequent cooling to the optimal survival temperature of the bacteria. The use of inactivated probiotics allows them to be incorporated in health forms of different sectors such as: nutraceuticals, dietetics, pharmaceuticals, cosmetics and foodstuffs itself; these probiotics do not require strict temperature and moisture control.

## Description

### OBJECT OF THE INVENTION

As the title states, the object of the invention is to define a series of health products containing inactivated probiotics, the use of the products with inactivated probiotics also being an object of the invention.

The present invention is characterised in that probiotics are provided in health mediums or products which have a moisture or temperature, either in their own right or by way of the manufacturing or storage process, at which common probiotics, which are untreated probiotics, would not stay alive.

Therefore, the present invention is framed within the scope of probiotics and health products or mediums in which the probiotics are provided.

### BACKGROUND OF THE INVENTION

Probiotics are live bacteria with activity that is beneficial for the human and/or animal organism. To date, administering probiotics in traditional pharmaceutical forms involves controlling temperature and moisture prior to, during and after the production of the products since the probiotics, as they are live bacteria, are sensitive to environmental conditions which are generally high temperatures and/or high moistures. If the suitable conditions of temperature and moisture (maximum temperature of 5°C and a moisture of less than 5% in the product) are not maintained during the storage, production and preservation processes, the probiotics may die and therefore lose efficacy and the beneficial interest for the human or animal organism.

Normal probiotics are defined by the World Health Organisation as live microorganisms which, when consumed in suitable quantities as part of food, provide the host with a health benefit. As said definition states, it is necessary for the bacteria to be live in order to exercise its activity. Probiotic bacteria are sensitive to high temperatures and moistures. They should not be subjected to temperatures greater than 5°C (their preservation below 0°C is recommended) and they should not contain moisture greater than 5% in order to be maintained live and/or to exercise their beneficial activity. Maintaining these conditions is very complicated for the sectors of nutraceuticals, dietetics, pharmaceuticals, cosmetics and foodstuffs both at the storage and production stage. The processes used to manufacture traditional pharmaceutical forms, foods and jelly mini-cups usually involve overcoming the optimal survival conditions of the probiotics which is why it is very easy to cause their death and therefore the finished product does not maintain the expected beneficial activity.

Given that there are numerous health products (nutraceuticals, dietetics, pharmaceuticals, cosmetics and foodstuffs) which do not meet the moisture and temperature conditions necessary to thereby provide probiotics which do not die or lose efficacy, the object of the present invention is to find the form in which the probiotics may be incorporated in the health products without losing efficacy or the beneficial ability which they exercise on the human organism, developing products such as those which are described below and the essentiality of which is reflected in the first claim.

### DESCRIPTION OF THE INVENTION

The object of the present invention is a series of health products which, while not meeting the conditions of maximum temperature of 5°C and moisture of less than 5%, in any step of storage, production and preservation allow probiotics to be incorporated without them dying or losing efficacy.

Said health products which have a temperature greater than 5°C and a moisture greater than 5% are characterised in that they incorporate inactivated or heat-killed probiotics.

Heat-killed probiotics are treated probiotics which do not require special or strict preservation conditions, except for the common conditions in other beneficial ingredients for human and/or animal health.

The inactivation of the probiotics is achieved by means of subjecting the probiotic strains to high temperatures (range 50°C to 140°C) for a time period of between 30 seconds and 90 minutes depending on the time of exposure of the bacteria to the temperatures, in various consecutive sessions and with subsequent cooling to the optimal survival temperature of the bacteria.

This process causes the probiotic strains to die, but they are not completely destroyed such that the bacteriocins which they contain are maintained active and the organic material of the bacteria (cell membrane, cytoplast, ribosomes, etc) are not destroyed which is why the organism (immune system) can recognise them as parts of a probiotic bacteria.

It has been proven that inactivated probiotics are beneficial for health to the same extent as normal probiotics. The action mechanism by way of which these inactivated probiotics are active is similar to the mechanism by way of which the normal probiotics act.

Owing to the characteristics of inactivated probiotics compared to traditional or live probiotics, they can be incorporated into health forms of different sectors such as: nutraceuticals, dietetics, pharmaceuticals, cosmetics and foodstuffs itself, these probiotics do not require strict temperature and moisture control.

The invention has application in liquid pharmaceutical forms, semi-solid forms and soft gelatine capsules as well as jelly mini-cups since these pharmaceutical forms require high concentrations of water and/or oil and/or high temperatures which makes the incorporation of traditional probiotics incompatible.

The use of inactivated probiotics in these sectors (nutraceuticals, dietetics, pharmaceuticals, cosmetics and foodstuffs itself) allows all the pharmaceutical forms, foods and jelly mini-cups to be manufactured, maintaining the specifications of the inactivated probiotics since it "cannot die" twice and therefore maintaining the beneficial properties of the products.

In summary, the use of the inactivated probiotics in the sectors described, allows health products to be manufactured (pharmaceuticals, cosmetics, foodstuffs and jelly mini-cups) guaranteeing the beneficial effect of the same due to the fact that they are capable of withstanding the technological manufacturing conditions. Traditional or normal probiotics cannot survive many of the technological processes which the forms of application described involve and therefore cannot guarantee the health benefits.

Unless otherwise indicated, all the technical and scientific elements used in this specification have the same meaning as a person skilled in the art belonging to this invention normally understands. Methods and materials similar or equivalent to those described in the specification may be used in the practice of the present invention.

Throughout the description and the claims, the word "comprises" and its variants do not intend to exclude other technical characteristics, additions, components or steps. Other objects, advantages and characteristics of the invention will emerge for a person skilled in the art in part from the description and in part from the practice of the invention.

### PREFERRED EMBODIMENT OF THE INVENTION

The invention of health products with inactivated probiotic products refers to health products of any sector such as nutraceuticals, dietetics, pharmaceuticals, cosmetics and foodstuffs itself which do not meet the conditions in order for common probiotics to be preserved since they have a degree of moisture greater than 5% and/or a temperature greater than 10°C in any of the processes of production, storage and preservation and into which inactivated probiotics have been incorporated.

The inactivation of probiotics, as has been previously indicated, is achieved by means of subjecting the probiotic strains to high temperatures (range 50°C to 140°C) for a time period of between 30 seconds and 90 minutes depending on the time of exposure of the bacteria to temperatures, in various consecutive sessions and with subsequent cooling to the optimal survival temperature of the bacteria.

Three examples of this invention are provided below.

To date, it has not been possible to use probiotics in syrups since the water content of the same makes the survival of the probiotic impossible.

Soft gelatine capsules, a widely used pharmaceutical form, cannot incorporate common probiotics since their high content of oil, water, etc does not allow the probiotic to survive.

Lastly, jellies cannot incorporate common probiotics in their interior since the production temperature as well as the water activity prevents their survival.

Therefore, owing to the use of the inactivated probiotics in different health-related sectors, health products can be manufactured (pharmaceuticals, cosmetics, foodstuffs and jelly mini-cups) guaranteeing the beneficial effect of the same due to the fact that they are capable of withstanding the technological manufacturing conditions. Traditional or normal probiotics cannot survive many of the technological processes which the forms of application described involve and therefore cannot guarantee the health benefits.

With the nature of the present invention sufficiently described as well as the manner of putting it into practice, it should be stated that, within its essentiality, it can be carried out in practice in other embodiments which differ in detail from that indicated by way of example and to which the protection sought similarly extends, provided its fundamental principle is not altered, changed or modified.

## Claims

1. Health products with a degree of moisture greater than 5% and/or a temperature greater than 5°C in any of the processes of production, storage and preservation, **characterised in that** inactivated probiotics have been incorporated into them.

2. The health products according to claim 1, **characterised in that** the inactivation of the probiotics is achieved by means of subjecting the probiotic strains to high temperatures (range 50°C to 140°C) for a time period of between 30 seconds and 90 minutes depending on the time of exposure of the bacteria to temperatures, in various consecutive sessions and with subsequent cooling to the optimal survival temperature of the bacteria.

3. A use of the products according to any of the preceding claims, **characterised in that** it is used in products from the nutraceutical sector.

4. A use of the products according to any of the preceding claims, **characterised in that** it is used in products from the dietetic sector.

5. A use of the products according to any of the preceding claims, **characterised in that** it is used in products from the pharmaceutical sector.

6. A use of the products according to any of the preceding claims, **characterised in that** it is used in products from the cosmetic sector.

7. A use of the products according to any of the preceding claims, **characterised in that** it is used in products from the foodstuff sector itself.
